# EUROPEAN PATENT SPECIFICATION

(11) **EP 0 259 983 B1**
(45) Date of publication and mention of the grant of the patent: **15.02.1995**
(21) Application number: 87307163.3
(22) Date of filing: 13.08.1987
(51) Int. Cl.: A61K 31/57, A61K 31/165, A61K 31/445, A61K 31/215

(54) **The use of corticosteroids for amelioration or elimination of postoperative pain**
Verwendung von Corticosteroide zur Verringerung von postoperativen Schmerzen
Utilisation des corticostéroids pour diminution ou élimination de la douleur postopératoire

(30) Priority: 27.08.1986 US 900683; 17.07.1987 US 74994
(43) Date of publication of application: 16.03.1988
(73) Proprietor: Cary Jr., George R., New Orleans Louisiana 70115 (US)
(72) Inventor: Cary Jr., George R., New Orleans Louisiana 70115 (US)
(74) Representative: Smart, Peter John

(56) References cited:
- THE JOURNAL OF FOOT SURGERY, vol. 22, no. 3, 1983, pages 187-191, The AmericanCollege of Foot Surgeons; G.A. CURDA et al.: "Postoperative analgesic effectsof dexamethasone sodium phosphate in bunion surgery"
- THE JOURNAL OF FOOT SURGEONS, vol. 24, no. 2, 1985, pages 142-147, The AmericanCollege of Foot Surgeons; C.F. FENTON et al.: "Postoperative intravenousdexamethasone administration; A preliminary investigation"
- ORAL SURG., vol. 40, 1975, pages 594-598; E.J. MESSER et al.: "The use of
- intraoral dexamethasone after extraction of mandibular third molars"
- BRITISH JOURNAL OF OPHTHALMOLOGY, vol. 64, 1980, pages 43-45; T.G. RAMSELL etal.: "Clinical evaluation of clobetasone butyrate: a comparative study of itseffects in postoperative inflammation and on intraocular pressure"
- DIALOG INFORMATION SERVICES, file 155: medline, accession no. 1321889; S.NIKONENKO et al.: "The effect ofhydrocortisone on healing of thepostoperative wound",& KHIRURGIIA (USSR),Sep. 1968, 44 (9) p131-2
- DIALOG INFORMATION SERVICES, file 155: medline, accession no. 4245049; R.M.KURBANOV: "Use of hydrocortisone intreating clean postoperative wounds",
- && KHIRURGIIA, (USSR), Nov. 1980,(11) p105-6

## Description

The present invention relates to the use of a corticosteroid in the preparation of a medicament for use in the treatment of post-operative pain.

Heretofore, the prior art has not disclosed an analgesic agent which is effective in reducing or eliminating postoperative pain without also producing detrimental side effect such as, for example drowsiness or nausea.

Among the objects of the present invention is the provision of a medicament which alleviates postoperative pain without such detrimental side effects. The foregoing as well as still further objects of the present invention will be more fully understood from the following description.

According to the present invention, the topical application of a cortisone preparation to the raw surface of a postoperative wound is useful in reducing or eliminating postoperative pain.

Accordingly, the present invention provides the use of a corticosteroid in the preparation of a medicament for use in the treatment of postoperative pain.

In accordance with the preferred procedure of the present invention, and following completion of an operative procedure, the raw surgical surfaces of the postoperative wound are doused once with the said cortisone prparation. A small amount of the material is applied to the surfaces of the wound in any convenient fashion, for example, by direct spray using an atomizer or by irrigation using a syringe and needle. The cortisone preparation is not irrigated out with saline following its application. The wound is closed in a routine fashion. Excess cortisone preparation drains out of the wound in the usual manner. Normal healing takes place.

Further, in accordance with the invention, the raw surgical surfaces of the postoperative wound can be treated with a cortisone preparation and a local anesthetic or other pain relieving medication.

It has been found helpful to the patient to apply a local anesthetic agent, preferably a long acting anesthetic agent, such as Marcain®, Xylocaine®, Lidocaine® or Novacaine® to the postoperative wound separately or along with the cortisone for immediate elimination of pain during the first several hours after the operation, the period of time that the cortisone is weakest in its effect.

The effect of the topical application of a cortisone preparation into the area of a postoperative wound, according to the invention, is dramatic. Postoperative pain is decreased significantly or eliminated. In cases of bilateral surgery when, for comparative purposes, one side is treated with a cortisone preparation while the other side is left untreated, the patient undergoes a painful postoperative recovery on the untreated side while experiencing no pain of significance on the treated side.

Any readily available cortisone preparation which is topically applicable can be used for present purposes. Celestone Suspension (sold as Celestone Soluspan® by Schering Corporation, Kenilworth, N.J.) is preferred, although other cortisone preparations which are sprayable or otherwise suitable for topical application may be used. Celestone® Suspension is a suspension brand of sterile beta-methasone sodium phosphate and beta-methasone acetate USP.

The use of beta-methasone and its derivatives, especially esters, is preferred.

The amount of cortisone applied to the surgical wound can be varied and will depend on such factors as the scope and severity of the wound. The effective amount of cortisone to be used can be readily ascertained for any specific situation.

Cortisone acts as an anti-inflammatory, reducing tissue swelling and thereby reducing painful pressure on surrounding nerves; in addition, cortisone acts directly on the severed ends of nerves. Research is being carried out to determine further the mechanism of action of cortisone when applied topically to surgical wounds.

The amount of local anesthetic or other pain relieving medication applied to the surgical wound can be varied and will depend on such factors as the scope and severity of the wound. The effective amount of anesthetic to be used can be readily ascertained for any specific situation.

The anesthetic can be applied to the surgical wound along with the cortisone or applied separately depending upon circumstances. Any readily available local anesthetic prepartion which is topically applicable can be used for present purposes. Representative examples of anesthetics that can be used include Marcain, Xylocaine, Lidocaine and Novacaine. The anesthetic can be applied to the surgical wound in any manner, for example, spraying, irrigating, sprinkling, or any appropriate mode available.

The present invention is illustrated in detail in the following examples.

### Example 1

### Bilateral Keller Bunionectomy

The soft tissue is excised over the medial aspect of the first MP joint of the great toe. The excision is continued through the soft tissues to the first joint of the great toe. The proximal phalanx of the great toe is removed, as well as the bunion over the medial portion of the distal aspect of the first metatarsal. Raw bony areas are left exposed, as well as the excised soft tissues.

A cortisone preparation, for example, Celestone® Suspension, is applied topically to the raw surgical surfaces of the wound, either by direct spray using an atomizer or by irrigation using a syringe and needle. When spraying, 1 mℓ of the cortisone preparation is applied to the raw surgical tissues; when irrigating, 1.5 mℓ to 2 mℓ are applied. The material is used on only one occasion. The cortisone preparation is not irrigated out with saline following its application. The wound is closed in a routine fashion. Excess cortisone preparation drains out of the wound by gravity. Normal healing takes place.

The patient commonly notes some discomfort in the recovery room when only cortisone is used. However, following recovery from the general anaesthetic, minimal discomfort in the surgical area is experienced. Utilizing pressure dressings over the feet, the patient is immediately able to ambulate about the room and otherwise.

When, for comparative purposes, one foot is treated with a cortisone preparation while the other foot is left untreated, the patient's response is dramatic. The patient complains significantly about the untreated foot and usually questions the surgeon as to what was done differently in the two extremities.

### Example 2

### Dupuytren's Excision of the Palm of the Hand

An excessive growth of subcutaneous fibrous tissue is removed from the palm of the hand, leaving a valley of exposed surgical tissues. The raw surfaces of the surgical tissues are doused once with, usually, less than 1 mℓ of a cortisone preparation, for example, Celestone Suspension or a combination of Marcaine and Celestone® mixed usually in equal volumes. The material is applied to the raw surgical surfaces of the wound either by direct spray using an atomizer or by irrigation using a syringe and needle. The cortisone preparation is not irrigated out with saline following its application. The wound is closed routinely. Excess cortisone preparation drains out of the wound in the usual manner. Normal healing takes place.

Although taking a bone graft from the ileum is a procedure which usually entails a painful postoperative recovery, the application of a cortisone preparation to the raw surgical surfaces of the postoperative wound eliminates significant postoperative discomfort in the area of the bone graft site.

The term "cortisone" used herein is not limited to, but includes 17 (β)-[1-keto-2hydroxyethyl]-Δ⁴androstene-3,11dione-17(α)ol, and embraces corticosteroids generally, especially those having glucocorticoid activity.

## Claims

1. The use of a corticosteroid in the preparation of a medicament for use in the treatment of post-operative pain by topical application of an effective amount thereof to raw surgical surfaces of a post-operative wound.

2. The use claimed in Claim 1, wherein the corticosteroid has glucocorticoid activity.

3. The use as claimed in Claim 1, wherein the corticosteroid is betamethasone or a derivative thereof.

4. The use claimed in Claim 3, wherein the corticosteroid is a suspension of a mixture of betamethasone acetate and betamethasone sodium phosphate.

5. The use as claimed in any preceding claim, wherein the medicament is filled into atomiser apparatus for use in application of the medicament by spraying directly on to wound surfaces.

6. The use claimed in any one of Claims 1 to 4, wherein the prepared medicament is suitable for application to raw surgical surfaces by irrigation using a syringe and needle.

7. The use claimed in any preceding claim, wherein the medicament further comprises a local anaesthetic.

8. The use claimed in Claim 7, wherein the local anaesthetic as Marcain, Xylocaine, Lidocaine, Novacaine or a mixture of two or more thereof.

## Patentansprüche

1. Verwendung eines Kortikosteroids bei der Herstellung eines Medikaments zur Verwendung bei der Behandlung postoperativer Schmerzen durch topische Applikation einer wirksamen Menge davon auf offene operative Oberflächen einer postoperativen Wunde.

2. Verwendung nach Anspruch 1, bei der das Kortikosteroid Glukokortikoid-Aktivität aufweist.

3. Verwendung nach Anspruch 1, bei der das Kortikosteroid β-Methason oder ein Derivat davon ist.

4. Verwendung nach Anspruch 3, bei der das Kortikosteroid eine Suspension einer Mischung von β-Methasonacetat und β-Methasonnatriumphosphat ist.

5. Verwendung nach einem der vorhergehenden Ansprüche, bei der das Medikament in einen Sprühapparat zur Verwendung bei der Applikation des Medikaments durch direktes Aufsprühen auf die Wundoberflächen gefüllt wird.

6. Verwendung nach einem der Ansprüche 1 bis 4, bei der das zubereitete Medikament geeignet ist zur Applikation auf offene operative Oberflächen durch Spülen unter Verwendung einer Spritze und einer Nadel.

7. Verwendung nach einem der vorhergehenden Ansprüche, bei der das Medikament ferner ein lokales Anästhetikum umfaßt.

8. Verwendung nach Anspruch 7, bei der das lokale Anästhetikum Marcain, Xylocain, Lidocain, Novacain oder eine Mischung von zwei oder mehreren derselben ist.

## Revendications

1. Utilisation d'un corticostéroïde dans la préparation d'un médicament destiné à servir au traitement de la douleur post-opératoire, par application topique d'une quantité efficace de ce médicament sur les surfaces chirurgicales vives d'une plaie post-opératoire.

2. Utilisation selon la revendication 1, dans laquelle le corticostéroïde a une activité de glucocorticoïde.

3. Utilisation selon la revendication 1, dans laquelle le corticostéroïde est la β-méthasone ou un de ses dérivés.

4. Utilisation selon la revendication 3, dans laquelle le corticostéroïde est une suspension d'un mélange d'acétate de β-méthasone et de phosphate sodique de β-méthasone.

5. Utilisation selon l'une quelconque des revendications précédentes, dans laquelle le médicament est versé dans un appareil d'atomisation pour servir à l'application du médicament par projection par pulvérisation directe sur les surfaces de la blessure ou de la plaie.

6. Utilisation selon l'une quelconque des revendications 1 à 4, dans laquelle le médicament préparé convient bien pour être appliqué, par une irrigation à l'aide d'une seringue et d'une aiguille, à des surfaces chirurgicales vives.

7. Utilisation selon l'une quelconque des revendications précédentes, dans laquelle le médicament comprend en outre un anesthésique local.

8. Utilisation selon la revendication 7, dans laquelle l'anesthésique local est du "Marcaïn", de la "Xylocaïne", de la "Lidocaïne", de la "Novocaïne" ou un mélange de deux ou plusieurs de ces anesthésiques.
